# EUROPEAN PATENT APPLICATION

(11) **EP 2 749 269 A1**
(43) Date of publication of application: **02.07.2014**
(21) Application number: 12199792.8
(22) Date of filing: 31.12.2012
(51) Int. Cl.: A61K 9/14, A61K 31/506, A61K 9/20

(54) **Process for the preparation of adsorbates of imatinib**

(71) Applicant: Deva Holding Anonim Sirketi, 34303 Istanbul (TR)
(72) Inventor: Koc, Fikret, 34303 ISTANBUL (TR); Karliga, Bekir, 34303 ISTANBUL (TR); Bellur Atici, Esen, 34303 ISTANBUL (TR); Ilhan, Suna Ayse, 34303 ISTANBUL (TR)

(57) **Abstract**

The present invention relates to a novel process for the preparation of lactose adsorbates of imatinib mesylate and/or its solvates or hydrates and pharmaceutical formulations prepared by employing adsorbates of imatinib mesylate.

## Description

### Technical Field

The present invention relates to adsorbates of imatinib mesylate and/or its solvates and a novel process for the preparation of or hydrates and imatinib mesylate adsorbates as well as pharmaceutical formulations prepared by employing said adsorbates.

### Background Art

Imatinib mesylate, is chemically 4-(4-methylpiperazin-1ylmethyl)-N (4-methyl-3-[4-pyrinin-3-yl) pyrimidin-2-yloamino] phenyl] benzamide mesylate, has a molecular formula of C₂₉H₃N₇O.CH₄SO₃ and a molecular weight of 589.7.

WO 95/09852 A (CIBA GEIGY AG) 13.04.1995 discloses firstly the preparation of imatinib as free base.

Imatinib is the first member of a new class of agents that act specially by inhibiting a certain enzyme that is characteristic of a particular cancer cell.

Imatinib is a protein-tyrosine kinase inhibitor, especially useful in the treatment of various types of cancer and can also be used for the treatment of atherosclerosis, thrombosis, restenosis, or fibrosis. Thus, imatinib can be also used for the treatment of non-malignant diseases.

Imatinib is usually administered orally in the form of a suitable salt, e.g., in the form of imatinib mesylate. The recommended dosage of imatinib is 400 mg per day for patients in chronic phase CML and 600 mg per day for patients in accelerated phase or blast crisis.

WO 99/03854 A (NOVARTIS-ERFINDUNGEN VERWALTUNGSGESELLSCHAFT MBH) 28.01.1998 discloses the methane sulphonic acid addition salt of imatinib and different polymorphic forms of imatinib, especially the alpha crystalline and beta crystalline forms of imatinib mesylate.

Polymorphism is defined as the ability of a substance to exist as two or more crystalline phases that have different arrangements or conformations of the molecules in the crystal lattice.

In this respect, there are many examples regarding to preparation of different polymorphic forms of imatinib mesylate in the literature.

The beta crystalline form is known to be its more compact crystalline form of imatinib mesylate which results in more beneficial flow properties and better processability in the manufacture of pharmaceutical preparations.

Its alternative, the alpha crystalline form of imatinib mesylate is characterized by needle-shaped crystals. Thus, the alpha crystalline form is not preferred by formulation scientist due to its unfavourable flow characteristic. High hygroscopicity of alpha crystalline form is a further disadvantage for processing and storing the acid addition salt in alpha form.

The flow properties of solids have great impact on the tableting of finished dosage form and the uniform flow of powders is one of the most important considerations in solid dosage manufacture. The improper feeding of powders from storage hoppers into dye-presses can lead to inconsistent product quality, causing economic and health impact. Hence, investigation of all properties affecting powder flow is crucial.

Different approaches have been taken lately to achieve a desired level of powder flowability of imatinib mesylate. But, none of the mentioned formulations/methods in the literature is completely satisfactory because they all demand technical effort and they are time and cost intensive. Thus, there is a constant need to develop a simple and economical process for manufacturing pharmaceutical formulations of imatinib mesylate not being restricted, to a particularly preferred morphology.

### Summary of invention

The present invention relates to adsorbates of imatinib mesylate and/or its solvates and a novel process for the preparation of or hydrates and imatinib mesylate adsorbates as well as pharmaceutical formulations prepared by employing said adsorbates. The present invention is further directed to an adsorbate of imatinib, comprising an adsorbent and imatinib on said adsorbent.

### Technical Problem

A compound may exist in several different crystal forms with different arrangements in the unit cell of its crystal in solid state and thus display different physical properties.

In this respect, when alpha crystalline form of imatinib is formulated with other ingredients, needle shape of said crystals causes lower flowability of the powder.

The uniform flow of powders is one of most important considerations in solid dosage manufacture. Weight, content uniformity, hardness and disintegration/dissolution of solid dosage form are known to be affected by formulation powder's flow. A continuous and uniform flow of powder from feeder to the die cavities is required during tablet manufacturing of solid dosage forms. Hence, pharmaceutical powders should be able to flow freely into storage containers or hoppers of tablet and capsule filing equipment so that a uniform packing of the particles and a uniform tablet or capsule weight is achieved.

In this respect, tablet pressing of high load, non needle shape of imatinib powder blend is very challenging, die and punch sticking becomes noticeable to an unacceptable degree.

Due to non homogeneous powder feeding to dies, weight variation occurs at high speed compression tablet.

Moreover, at low speed compression, longer dwell time may cause the substance to melt as the heat resulting from compression regarding to the difficulties in tableting.

Some polymorphic forms of imatinib mesylate, especially alpha form is not freely flowing which present problems in development and production of all solid dosage forms.

The above conditions are limiting the pharmaceutical formulation studies of imatinib mesylate. Thus, a manufacturing method which is free from characteristic of a particular crystal form is needed.

It is an objective of the present invention to further optimize tablet manufacturing containing imatinib mesylate by not being restricted, to a particularly preferred morphology.

The high dosage administered to patients and the problems reported regarding the difficulties in tableting, it is an objective of the present invention to further optimize imatinib tablet manufacturing process.

### Solution to Problem

The inventors of the present invention have now found that the flowability of imatinib might be satisfactorily improved by adsorbate formation. Even needle shaped alpha crystalline form's bad compaction behaviour is improved and better flowability and tableting properties are obtained.

In the present invention, inventors disclose a process for preparing an adsorbate which comprises an adsorbent and imatinib adsorbed on said adsorbent.

The process according to the invention yields surprisingly homogeneous adsorbates of imatinib. These imatinib adsorbates are used as active pharmaceutical ingredient in preparations.

The adsorbate of imatinib mesylate of the present invention has found to possess good flowability and compressibility features.

The solid pharmaceutical formulations obtained according to the present invention as adsorbates do not need any additional step or excipient and may be prepared starting from any particle size of imatinib.

Also, inventors optimized related process parameters which affect critical flow properties.

### Brief description of drawings

Fig.1 provides the scanning electron microscopy of alpha crystalline form of imatinib mesylate.

Fig.2 provides the scanning electron microscopy of bulk lactose.

Fig.3 provides the scanning electron microscopy of lactose adsorbed imatinib mesylate.

Fig.4 provides the scanning electron microscopy of physical mixture of imatinib mesylate and lactose.

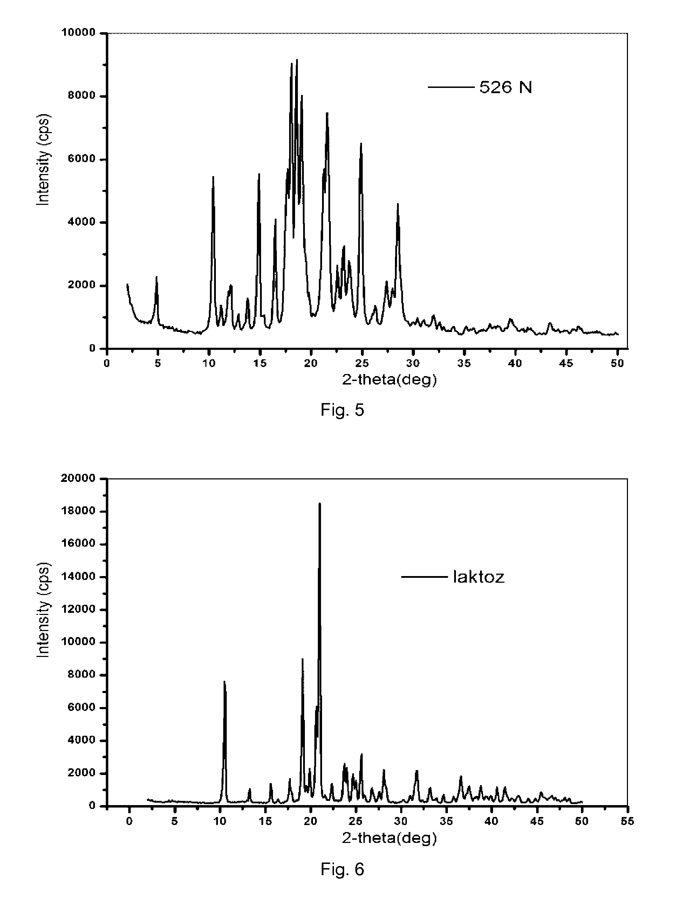

Fig.5 provides the X-ray diffractogram of the alpha crystalline form of imatinib mesylate.

Fig.6 provides the X-ray diffractogram of bulk lactose.

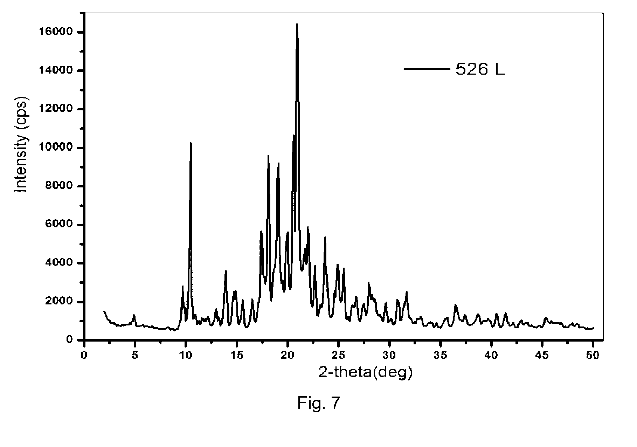

Fig.7 provides the X-ray diffractogram of lactose adsorbed imatinib mesylate.

### Description of embodiments

The main objective of the present invention is to provide a simple and economical process for the preparation of imatinib mesylate not being restricted to a particularly preferred morphology and that can be used immediately for manufacturing pharmaceutical formulations.

Aspects of the present invention relate to adsorbates of imatinib mesylate and pharmaceutical formulations comprising these adsorbates.

In a first embodiment of the present invention, a process for preparing an adsorbate which comprises an adsorbent and imatinib adsorbed on said adsorbent.

The invention discloses a process for the preparation of adsorbates of imatinib mesylate and/or of its hydrates comprises a) dissolving imatinib free base in the solvent to obtain solution b) adding an adsorbent to the solution prepared in step(a) and c)recovering the adsorbate from the mixture of step(b).

The solution of imatinib is prepared by dissolving imatinib in the solvent to obtain said solution and then adsorbent is suspended in said solution.

Organic solvents in which the active pharmaceutical ingredient will be dissolved are suitable for the process according to invention, for the preparation of imatinib adsorbates.

The suspension of said adsorbent in a solution of imatinib can be prepared by either suspending said adsorbent in said solution or dissolving imatinib in a suspension of said adsorbent.

In the adsorbates of the present invention, the adsorbent may preferably be selected from cellulose, cellulose derivatives, polyols, sugars, sugar alcohols and other sugar derivatives, starches, pre-gelatinized starches, starch derivatives, modified starches, dextrins, maltodextrins, polydextroses, dextroses, inorganic excipients and mixtures thereof. As inorganic excipients calcium carbonate, calcium phosphate, calcium sulphate and mixtures thereof may be exemplified. Preferred adsorbents are microcrystalline cellulose, lactose, mannitol, starch, pregelatinized starch and calcium phosphate.

In an advantageous embodiment of the present invention, the adsorbent is selected from direct compressible excipients known to the skilled person.

In the present invention, a direct compressible excipients sugars, polyols, starch products and mixtures thereof to prepare an adsorbate of the present invention.

In the present invention, inventors preferred lactose which is used as adsorbent due to its low hygroscopicity, good compatibility with active ingredient and other excipients. Excellent physical and chemical stability and water solubility characteristics of lactose are further advantages of lactose in the use of adsorbates.

In a second embodiment of the present invention, adsorbates of imatinib mesylate and/or its solvates or hydrates that are obtainable by said process and physical properties of these adsorbates are disclosed.

The ratio of pharmaceutical active ingredient to adsorbing material is in the range from 1:0.1 to 1:10, a range from 1:0.5 to 1:2 being particularly preferred.

In another embodiment of the present invention, the adsorbates of imatinib mesylate which have shown superior flow properties are disclosed. These adsorbates are highly suitable in all formulations of imatinib mesylate.

Powder flowability is measured using the Hausner ratio, the carr index and angle of repose. Moreover, some parameters such as compressibility index are most commonly used parameters in the characterization of powder flowability.

While tapped and untapped density is crucial to characterize flow properties of imatinib mesylate, compressibility index is indirect measure of the flowability of the powder and it is determined according to US Pharmacopeia General Chapter <1174>.

In a further aspect, the present invention relates to pharmaceutical powder including adsorbates of imatinib mesylate having compressibility index <20.

According to preceding claims, a) particles of adsorbates of imatinib mesylate and/or its solvates or hydrates, b) powder mixtures of adsorbates of imatinib mesylate and/or its solvates or hydrates with an excipient or mixtures of excipients are characterized by that compressibility index of particles is not greater than 20.

According to obtained results of batches, final blend of lactose adsorbates of imatinib mesylate and/or its solvates or hydrates have an average of compressibility index of 12.50.

**Table 1**

| | Bulk Density | Tapped Density | Hausner Ratio(TD/BD) | Compress ibility Index (CI) |
|---|---|---|---|---|
| Alpha form imatinib mesylate | 0,52 | 0,648 | 1,13 | 19,77 |
| Microcryst alline cellulose adsorbate of alpha form imatinib mesylate | 0,482 | 0,556 | 1,15 | 13,33 |
| Lactose Adsorbate of alpha form imatinib mesylate | 0,471 | 0,538 | 1,14 | 12,5 |

Flow characteristics of granules and disintegration and dissolution of the finished dosage form containing adsorbates of imatinib mesylate are particularly dependent on morphology.

The lactose adsorbate may be incorporated into a pharmaceutical composition for oral administration using one or more pharmaceutically acceptable excipients.

In the present invention, pharmaceutical composition comprising adsorbates of imatinib mesylate and/or its solvates or hydrates can contain one or more excipients, such as diluents, binders, disintegrants, lubricants.

The amount of each type of additive employed, e.g. glidant, binder, disintegrant, filler or diluent and lubricant may vary within conventional ranges in the art.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, a-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches ( including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A binder is used to impart cohesive qualities to the solid dosage form and thus ensure that a tablet remains intact after compression. Examples of binders include, but not limited to, microcrystalline cellulose, hydroxymethyl cellulose, hydroxypropylcellulose, starch (including corn starch and pregelatinized starch), gelatin, sugars (including sucrose, glucose, dextrose, lactose, and sorbitol), waxes, polyethylene glycol, natural and synthetic gums (e.g.acacia, tragacanth sodium alginate, celluloses, and Veegum),and synthetic polymers such as polymetacrylates and polyvinylpyrrolidone (povidone), ethylcellulose, hydroxyethyl cellulose, polyethylene oxide, mixtures thereof and the like.

A disintegrant is a substance which helps the composition break up once ingested. Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, microfine cellulose, low-substituted hydroxypropylcellulose, mixtures thereof and the like.

Glidants improve the flowability of the composition. The composition may also comprise a glidant. Glidants are, but not limited to, colloidal silica, powdered cellulose, talc, tribasic calcium phosphate, mixtures thereof and the like.

The presence of a lubricant is particularly preferred when the composition is a tablet to improve the tableting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

The amount of each type of additive employed, e.g. glidant, binder, disintegrant, filler or diluent and lubricant may vary within ranges conventional in the art.

Selection of excipients and amounts to use can be determined by the scientist. One or more these additives can be chosen and used by the artisan having regard to the particular desired properties of the solid dosage form.

For an improvement of flow properties, additives containing silica or titania can be used.

In another embodiment of the present invention, the solid pharmaceutical formulations of these imatinib mesylate adsorbates exhibit improved dissolution characteristics. In another aspect of this invention, adsorbates of imatinib mesylate are evaluated by X-ray diffraction diagrams and scanning electron microscopy.

X-ray diffraction data were acquired using a Rigaku smart X-ray diffractometer model SmartLab. System description: K_{α1}=1.54178Å, voltage 40 kV, current 30 mA, incident slit1/2°, receiving slit=0.2mm, scattering slit=1° with a Graphite monochromator. Measurements of 2θ values typically are accurate to within ±0.2 degrees. Experiment parameters: pattern measured between 2θ=3° and 2θ=50° with 0.05° increments; count time was 0.5 second per increment.

Electron-micrographs of adsorbates are obtained using a scanning electron microscope. Adsorbates of imatinib mesylate and/or its solvates are identified with Scanning Electron Microscope (SEM-JCM -5000 NeoScope model).

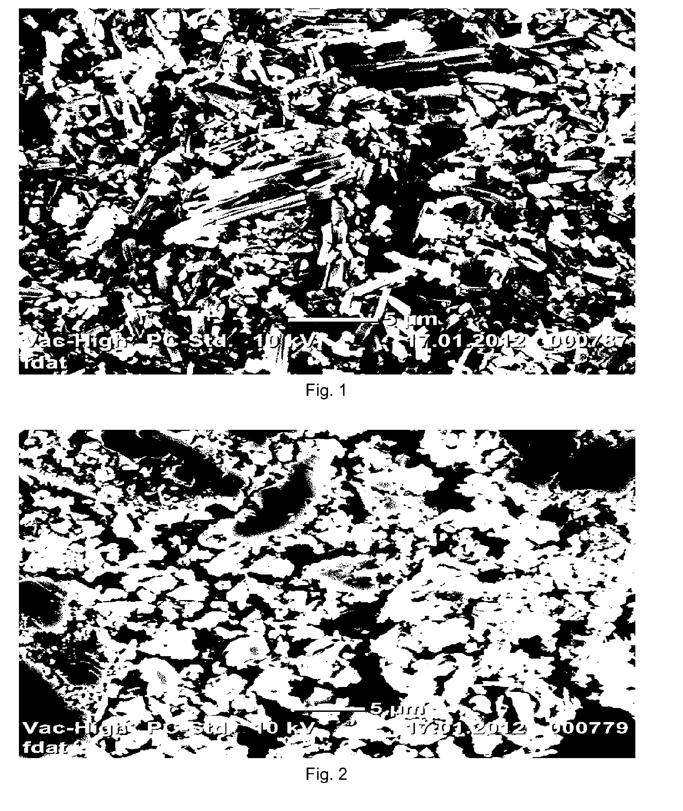

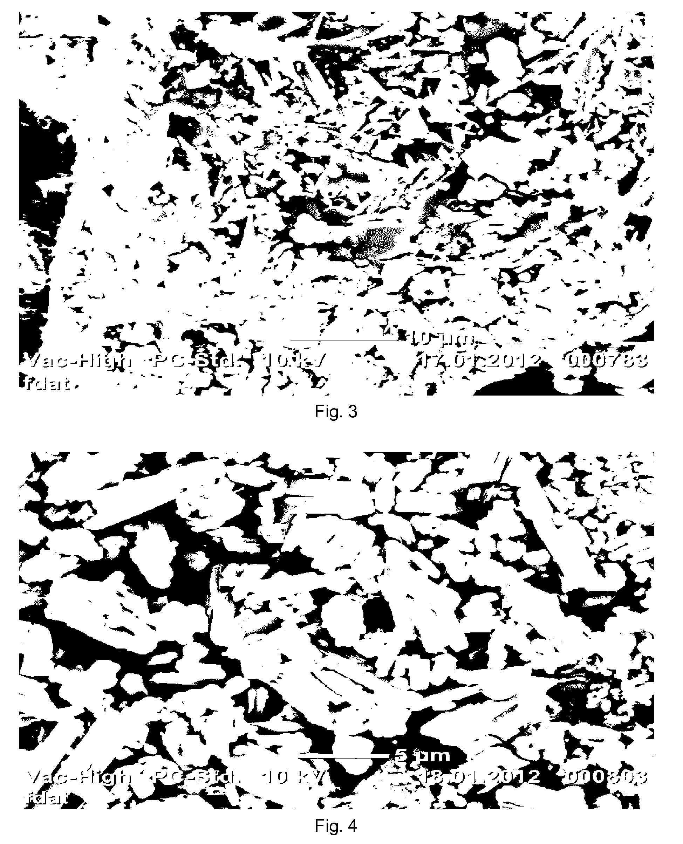

The alpha crystalline form of imatinib mesylate and bulk lactose have the SEM pattern shown respectively in Fig.1 and Fig.2. The obtained lactose adsorbed imatinib mesylate obtained has the SEM pattern shown in Fig.3.

The improvement of flow properties of powder is also observed in Fig.3.

The physical mixtures were prepared by mixing imatinib mesylate and lactose with a test tube mixer for 5 min at a constant rate. These samples were passed through an appropriate sieve.

Inventors of the present invention have clearly noticed that the flow properties of lactose adsorbates of imatinib mesylate and physical mixture of lactose and imatinib are clearly different from each other.

The following examples are provided to further illustrate the adsorbates, pharmaceutical compositions, and manufacturing processes of the present invention. Given examples are illustrative and not intended to limit the scope of the invention.

### Example:Preparation of Lactose Adsorbates of Imatinib Mesylate

Into the reactor a suspension of 85 ml of THF and 3 gr of imatinib base is charged and stirred at 60°C to get clear solution. 2.02 gr lactose is added to the reactor and stirred further for 30 minutes. 0,395 gr methane sulphonic acid is dissolved in 50 ml THF. The methane sulphonic acid solution is added to the reaction mixture slowly during 30 minutes. The reaction mixture is stirred for another 30 minutes at 65°C and cooled 30°C. The formed crystals are filtered and dried under vacuum for about 6 hours at 50°C.

### Example:Pharmaceutical Formulation

**Table 2**

| **PERCENTAGES BASED ON BY WEIGHT OF TOTAL PHARMACEUTICAL COMPOSITION** | | |
|---|---|---|
| | **mg/tablet** | **%(w/w)** |
| **Ingredients** | | |
| Lactose Adsorbate of Imatinib Mesylate* | 478.0 | 33.31 |
| Microcrystalline Cellulose(PH 101) | 230.0 | 16.02 |
| Hydroxypropyl methylcellulose (HPMC) | 35.0 | 2.44 |
| Microcrystalline Cellulose(PH 102) | 373.0 | 26.00 |
| Crospovidone | 259.5 | 18.08 |
| Colloidal Silicon Dioxide | 11.0 | 0.77 |
| Magnesium Stearate | 13.5 | 0.94 |
| Water** | q.s. | |
| **Total Core Tablet Weight** | **1400.0** | **97.56** |
| Polyvinyl alcohol | 14.00 | 0.97 |
| Polyethylene glycol | 7.07 | 0.49 |
| (Macrogol)/PEG 3350 | | |
| Yellow iron oxide | 6.65 | 0.46 |
| Talc | 5.18 | 0.37 |
| Titanium Dioxide | 1.75 | 0.12 |
| Red Iron Oxide | 0.35 | 0.03 |
| Water | q.s. | |
| **Total Film Coating Weight** | **35.00** | **2.44** |
| **Total Tablet Weight** | **1435.00** | **100.00** |

| | | |
|---|---|---|
| *containing 400 mg of imatinib free base equivalent. | | |

The tablets are prepared by wet granulation of imatinib mesylate, microcrystalline cellulose, hydroxypropyl methyl cellulose, crospovidone, colloidal silicon dioxide with water. Then resultant tablets are compressed and coated with an aqueous dispersion of the coating mixture.

Tablets prepared by using adsorbates of imatinib mesylate have low friability, good surface finish and are less prone to abrasion.

**Table 3**

| Time (minutes) | Dissolved (%) |
|---|---|
| 10 | 92.4 |
| 15 | 94.3 |
| 20 | 95.1 |
| 30 | 96.0 |
| 45 | 96.8 |
| 60 | 97.5 |

## Claims

1. A pharmaceutical composition comprising an adsorbate of imatinib mesylate and/or of its hydrates and optionally one or more pharmaceutically acceptable ingredients.

2. According to claim 1, the pharmaceutical composition **characterizing in that** imatinib adsorbed on a pharmaceutically acceptable adsorbent, optionally along with one or more pharmaceutically acceptable excipients.

3. According to claim 1, a process for the preparation of adsorbates of imatinib mesylate and/or of its hydrates comprises a) dissolving imatinib free base in the solvent to obtain solution b) adding an adsorbent to the solution prepared in step (a) and c) recovering the adsorbate from the mixture of step(b).

4. According to claim 1, adsorbent is selected from the group consisting of cellulose, cellulose derivatives, polyols, sugars, sugar alcohols and other sugar derivatives, starches, pre-gelatinized starches, starch derivatives, modified starches, dextrins, maltodextrins, polydextroses, dextroses, calcium carbonate, calcium phosphate, calcium sulphate and mixtures thereof, more preferably microcrystalline cellulose, lactose, mannitol, starch, pregelatinized starch and calcium phosphate.

5. According to claim 1, the ratio of imatinib to adsorbing material is in the range from 1:0.1 to 1:10, preferably in the range from 1:0.5 to 1:2.

6. According to claim 1, powder mixtures of adsorbates of imatinib mesylate and/or its solvates or hydrates with an excipient or mixtures of excipients **characterizing in that** that compressibility index of particles is not greater than 20.
